# EUROPEAN PATENT SPECIFICATION

(11) **EP 4 284 230 B1**
(45) Date of publication and mention of the grant of the patent: **19.02.2025**
(21) Application number: 21731407.9
(22) Date of filing: 27.05.2021
(51) Int. Cl.: A61B 3/117, A61F 9/007, A61N 5/06, A61B 3/13, A61B 3/00

(54) **APPARATUS AND SYSTEMS FOR MONITORING AND TREATING CATARACTS**
VORRICHTUNGEN UND SYSTEME ZUR ÜBERWACHUNG UND BEHANDLUNG VON KATARAKT
APPAREILS ET SYSTEMES DE SURVEILLANCE ET DE TRAITEMENT DES CATARACTES

(30) Priority: 26.01.2021 GB 202101007
(43) Date of publication of application: 06.12.2023
(73) Proprietor: Edinburgh Biosciences Limited, Livingstone EH54 7DP (GB)
(72) Inventor: CRUICKSHANK, Calum, Kirkton Campus Livingston EH54 7DP (GB); KERR, Alan, Kirkton Campus Livingston EH54 7DP (GB); SHAHID, Shahjahan, Kirkton Campus Livingston EH54 7DP (GB); SHARP, Andrew, Kirkton Campus Livingston EH54 7DP (GB); SMITH, Desmond, Kirkton Campus Livingston EH54 7DP (GB)
(74) Representative: Duffy, Claudia Magdalena
(86) International application number: PCT/EP2021/064249
(87) International publication number: WO 2022/161647

(56) References cited:
- WO-A1-2015/131135
- US-A1- 2014 104 576
- US-A1- 2018 353 769
- US-B2- 9 220 403
- ANNA GAKAMSKY ET AL: "Tryptophan and Non-Tryptophan Fluorescence of the Eye Lens Proteins Provides Diagnostics of Cataract at the Molecular Level", SCIENTIFIC REPORTS, vol. 7, 10 January 2017 (2017-01-10), pages 40375, XP055389271, DOI: 10.1038/srep40375
- BUTTENSCHOEN KIM K ET AL: "Measurement and quantification of fluorescent changes in ocular tissue using a novel confocal instrument", PROCEEDINGS OF SPIE, IEEE, US, vol. 9129, 8 May 2014 (2014-05-08), pages 91291R - 91291R, XP060038838, ISBN: 978-1-62841-730-2, DOI: 10.1117/12.2051800

## Description

### FIELD OF THE INVENTION

This invention relates generally to the field of non-invasive cataract treatments, in particular, to cataract treatments using treating light of selected wavelengths. More particularly, the invention relates to an apparatus for monitoring and treating cataracts using a monitoring light source and a treating light source. Furthermore, the invention relates to systems for use with and/or within an apparatus for monitoring and treating cataracts.

### BACKGROUND OF THE INVENTION

A *cataract* is an opacification (a clouding) of the crystalline lens of the eye due to metabolic changes of the crystalline lens fibres over time. The clouding may develop in the crystalline lens of the eye or in its envelope and it varies in degree from slight to complete opacity and obstructs the passage of light.

The yellow coloration of the lens accompanying the cataract is believed to be caused by the formation of covalent cross-links and aggregation of degraded proteins in the lens. Covalent cross-links and other types of degradation disrupt the optical and mechanical properties of the lens. The cross-links may be Sulphur bridges occurring between and/or within the proteins of the lens. The fluorescence of cyclic molecular components of the cross-links is early evidence of this process. Thus, a cataract manifests itself as a protein conformational disease characterised by accumulation of light absorbing, fluorescent and scattering protein aggregates.

The emergence of a cataract causes impairment or loss of vision. Cataracts often develop slowly and can affect one or both eyes. Symptoms may include faded colours, blurry or double vision, halos around light, trouble with bright lights, and trouble seeing at night. This may result in trouble driving, reading or recognising faces.

There are no currently known means of preventing cataracts and the only available treatment is by *invasive surgery. Cataract surgery,* also called *lens replacement surgery,* is the removal of the natural lens of the eye that has developed an opacification and its replacement with an artificial lens (i.e., an intraocular lens). The artificial lens is positioned in the same place as the natural lens, thus it remains a permanent part of the eye.

To date, cataract surgery is the only effective treatment. Cataract surgery is generally safe, but it carries a risk of infection and bleeding. Furthermore, serious complications of cataract surgery include retinal detachment and endophthalmitis. In both cases, patients notice a sudden decrease in vision. In endophthalmitis, patients often describe pain. Retinal detachment frequently presents with unilateral visual field defects, blurring of vision, flashes of light or floating spots.

Although cataract surgery is the only effective treatment option, it remains unavailable in sufficient quantity for the vast majority of the world population living in areas without access to specialised health care. Therefore, reducing vision impairment or blindness due to cataracts requires solutions that can be applied outside operating theatres.

There is a need for a *non-invasive cataract treatment* apparatus able to treat cataracts without the need to remove and replace the eye's natural lens, thus without the need of using an operating theatre.

US2011202114A1 (Kessel et al.) discloses a light-based non-invasive cataract treatment method which is said to avoid or postpone the need for cataract surgery by ten to possibly thirty years. The method is based on a non-invasive light therapy where the age-related protein changes of the cataractic lens are reversed by radiation with laser light. Human donor lenses were shown to be treated (by photo-bleaching) with an 800 nm infra-red femtosecond pulsed laser in a treatment zone measuring 1x1x0.52 mm. After laser treatment, the age-induced yellow discoloration of the cataractic lens was markedly reduced and the transmission of light was increased, corresponding to an optical rejuvenation of 3 to 7 years.

The drawback of the light-based non-invasive cataract treatment method disclosed in US2011202114A1 is that femtosecond pulsed lasers increase the complexity and the risk factor of the light-treatment apparatus, thus making the apparatus costly to maintain and hence more costs to doctors and hospital management. Also, the method only appears to delay the onset of cataracts, rather than treat them, and the treated volume is very small. Other cataract treatment systems are disclosed in US 2018/353769 A1, US 9220403 A2 or US 2014/104576 A1, for example.

Furthermore, a study published on 7-June-2020 in the European Journal of Ophthalmology (https://journals.sagepub.com/doi/abs/10.1177/1120672120922448) on the 'The benefits and drawbacks of femtosecond laser-assisted cataract surgery' suggests that a 'Femtosecond laser-assisted cataract surgery seems to be beneficial in some groups of patients, that is, with low baseline endothelial cell count, or those planning to receive multifocal intraocular lens. Nevertheless, having considered that the advantages of femtosecond laser-assisted cataract surgery might not be clear in every routine case, *it cannot be considered* as *cost-effective'.*

Thus, there is a need for a light-based cataract treatment apparatus which is *safe to use and not expensive* (i.e., an apparatus which is reasonably priced and which can be safely used in a main-stream opticians shop), *cures cataracts* (rather than delaying the need for cataract surgery) and is able to *expose a larger volume of the eye's lens* to the treating light (i.e., an area as large as the area covered by a standard slit-lamp microscope routinely used in main-stream opticians shops). Such cataract treatment apparatus retains the eye's natural lens, avoids the need for invasive surgery and can be deployed in the community, improving availability and accessibility to cataract monitoring and treatment.

The skilled person would be guided by the following glossary of terms which, within the context of the present invention, should be taken to mean:
- absorption - a photon being taken up by an atom or molecule and causing a change in the state of an electron in the atom, typically followed by radiative decay (fluorescence, phosphorescence etc) or thermal relaxation.
- aqueous humour - a watery fluid produced by the eye, in front of the crystalline lens; it provides nutrition to the eye and maintains pressure with the eye.
- beam splitters - in fluorescence microscopy, dichroic filters are used as beam splitters to direct illumination of an excitation frequency toward the sample and then at an analyser to reject that same excitation frequency but pass a particular emission frequency.
- crystalline eye lens - the natural lens that focusses light onto the back of the eye.
- cornea - transparent part at the front of the eye, contributes to the focussing of light.
- dichroic filter (or thin-film filter or interference filter) - a very accurate colour filter used to selectively pass light of a small range of colours while reflecting other colours; the interference is produced by alternating layers of optical coatings with different refractive indices built upon a glass substrate; the layers are usually added by vacuum deposition; by controlling the thickness and number of the layers, the frequency of the passband of the filter can be tuned and made as wide or narrow as desired.
- fluorescence emission - a luminescence in which the molecular absorption of a photon triggers the emission of another photon with a longer wavelength.
- fluorescence microscope - an optical microscope that uses fluorescence instead of, or in addition to, scattering, reflection, and attenuation or absorption, to study the properties of organic or inorganic substances.
- lock-in amplifier - a type of amplifier that can extract a signal with a known frequency from an extremely noisy environment.
- monitoring light - light of selected wavelengths emitted by a monitoring LED (light emitting diode); also referred to as the cataract 'excitation light'.
- monitoring mode - exposure of the eye's lens to monitoring light emitted by an LED at selected power.
- NFK - N-Formyl Kynurenine; a degradation product of tryptophan.
- phase sensitive detector - compares the phases of the reference and incoming signal and is thus able to recover a weak signal from an overwhelming background noise.
- photo-bleaching - use of light to reverse protein aggregation.
- PMT - photo-multiplier tube detector
- PSD - phase sensitive detection
- ratio scan - scan of the eye's lens using a pre-selected wavelength of the monitoring light and only 2 wavelengths of the cataract fluorescence emission.
- reflection - specular reflection (i.e. the throwing back of light at a surface that follows the laws of reflection and that does not randomise the polarisation of a photon of light).
- retina - thin film at the back of the eye that converts light into neural impulses.
- scattering - Rayleigh scattering, Mie scattering, Raman scattering, diffuse reflection, etc that randomises the direction and polarisation of a photon of light.
- scattering angle - the angle between the direction of propagation of light before and after a scattering event.
- spectral scan - scan of the eye's lens using a pre-selected wavelength of the monitoring light and a wavelength spectrum of the cataract fluorescence emission.
- treating light - light of selected wavelengths emitted by a treating LED.
- treatment mode - exposure of the eye's lens to treating light emitted by an LED at selected power.
- treatment - non-invasive photo-bleaching of the eye's lens using treating light emitted by an LED at a pre-selected treating wavelength.
- Tryptophan - a fluorescent amino acid typically found in proteins in the crystalline lens.
- vitreous humour - like aqueous humour, except that it is found behind the crystalline lens.

### PROBLEM TO BE SOLVED BY THE INVENTION

There is a need for an *apparatus* able to monitor and treat cataracts using monitoring and treating light sources. The monitoring and treating can be performed sequentially or simultaneously. There is also a need for *systems* for use with and/or within an apparatus for monitoring and treating cataracts. Such systems would be ones whereby the apparatus for monitoring and treating cataracts can become an attachment to existing ophthalmic instruments (e.g. a slit-lamp microscope), rather than a stand-alone apparatus. Other systems would be ones which can be integrated within the apparatus for monitoring and treating cataracts to enable accurate selection of fluorescence wavelengths emitted by cataracts during monitoring and/or treatment.

Therefore, it is an object of the present invention to provide an *apparatus for monitoring and treating cataracts,* the apparatus having a defined focus on the monitoring and the treatment of cataracts using light sources, whereby the monitoring light, the treating light and the excited fluorescence light are reflected along a common optical axis between the eye and a device for detecting fluorescence emitted by monitored and/or treated cataracts.

It is a further object of this invention to provide *systems* for use *with* and/or *within* an apparatus for monitoring and treating cataracts. A *wavelength selection system* may be incorporated within the apparatus for monitoring and treating cataracts to enable the apparatus to accurately measure the fluorescence of two fluorescent bands characteristic of cataracts emission. Such a system would allow the apparatus to have a good emission light to noise ratio from the cataract fluorescence. An *apparatus control system* comprising an electronic device may be used with the apparatus for monitoring and treating cataracts to enable, for example, the selection of an operating mode of the apparatus. The operating mode of the apparatus may be selected from (i) a *monitoring mode* when the electronic device manages and controls the power supply and the exposure time of a monitoring light source or (ii) a *treatment mode* when the electronic device manages and controls the power supply and the exposure time of a treating light source.

### SUMMARY OF THE INVENTION

The invention is defined in claim 1, claim 16 and claim 21. Further aspects and preferred embodiments are defined in the appended claims. Aspects, embodiments and examples of the present disclosure which do not fall under the scope of the appended claims do not form part of the invention and are merely provided for illustrative purposes.

In accordance with a *first aspect of the invention,* there is provided an apparatus for monitoring and treating cataracts, the apparatus comprising: a monitoring light source configured to monitor cataracts by emitting monitoring light in the wavelength range of 350 to 410 nm to excite fluorescence light in the cataracts, a treating light source configured to treat cataracts by emitting treating light in the wavelength range of 400 to 570 nm to irradiate the cataracts, a wavelength selection system configured to monitor cataracts by selecting wavelengths of the excited fluorescence light in the cataracts and a dichroic beam splitter configured to reflect the monitoring light and the treating light towards the cataracts and the excited fluorescence light in the cataracts towards the wavelength selection system, *wherein* the monitoring light, the treating light and the excited fluorescence light are reflected by the dichroic beam splitter along a common optical axis and *wherein* the dichroic beam splitter is arranged at 45 degrees to the common optical axis to transmit wavelengths longer than wavelengths of the monitoring light, the treating light and the excited fluorescence light towards a user of the apparatus.

The advantage of having a dichroic beam splitter arranged at 45 degrees to the common optical axis is that the apparatus for monitoring and treating cataracts can become an attachment to existing ophthalmic instruments (e.g. a slit-lamp microscope, such as a Keeler slip lamp https://www.keeler.co.uk/products/slit-lamps.html). The dichroic beam splitter is thus allowing long wavelength visible light to pass through to the slit-lamp microscope so that a visual (or camera) check on the positioning of the patient's eye can be maintained by the operator of the apparatus. This configuration in turn enables the use of a computerised tracking system using the image captured by the camera built into the slit- lamp microscope. The tracking system can alert the operator when the patient's eye moves out of position, thus allowing better control of the treated area and the actual treatment dose provided to a patient's eye.

Furthermore, the dichroic beam splitter ensures that the monitoring light, the treating light and the excited fluorescence light are reflected along a common optical axis, thus enabling the apparatus to simultaneously monitor and treat cataracts and also minimise optical loses.

The monitoring light source may comprise a non-lasing LED light source operable to emit light in the wavelength range of 350 to 410 nm, preferably in the wavelength range of 360 to 370 nm and more preferably at 365 nm to excite fluorescence light in the cataracts. The use of a 365 nm monitoring (or excitation) light allows adequate penetration through the cornea into the eye and transmission of the resulting fluorescence emission spectra out of the eye. Wavelengths down to about 350 nm would also produce cataract fluorescence spectra, but would require higher excitation powers. Excitation wavelengths up to about 410 nm would provide better penetration of the cornea but reduce the information from the resulting fluorescence spectra.

The treating light source may comprise a non-lasing LED light source operable to emit light in the wavelength range of 400 to 570 nm, preferably in the wavelength range of 410 to 420 nm and more preferably at 415 nm to irradiate the cataracts. The use of a 415 nm treating light makes the cataract irradiation a truly non-invasive in-vivo treatment since it allows the treating light to be focused on the lens to protect the retina.

The wavelength selection system may comprise any one or a combination of any one of a linear variable interference filter, a diffraction grating and a refractive prism. The advantage of such optical elements permits spectral imaging of targeted regions within the lens of a patient's eye, therefore allowing environmentally broadened emissions of tryptophan and NFK to be recorded without distortion.

Preferably, the linear variable interference filter may comprise a tuneable bandpass interference filter operable in the wavelength range of 320 to 560 nm. The advantage of the chosen bandwidth is that the bandpass interference filter allows measuring the natural broadening of 10 nm bandwidth of the excitation spectra, thus optimising capturing the cataractic protein fluorescence.

More preferably, the tuneable bandpass interference filter may comprise a wedge filter. The advantage of using a wedge filter is that it allows recording non-normal light incidence, which in turn allows a high light grasp, thus making it possible to spectrally analyse all the fluorescence emission from the target cataractic eye.

The wavelength selection system may further comprise a *linear drive operable to move the linear variable interference filter along an axis perpendicular* to the common optical axis. The advantage of the linear drive moveable perpendicular to the common optical axis is that it enables the filter to be moved accurately to each nm of wavelength for capturing a wide range of wavelengths of the fluorescence spectrum of a cataractic eye lens.

Preferably, the apparatus may further comprise *a detector,* more preferably a single detector. Routine optics may be designed to capture the fluorescence signal and direct it onto the filter before the transmitted portion is focused onto the detector. The advantage of using a combination of a linear variable interference filter and a detector is that the filter can be moved (by the linear drive) in suitable step sizes across the detector. Therefore, this optical arrangement of the apparatus can record fluorescence spectra one wavelength at a time.

More preferably, the detector may comprise a photo-multiplier tube detector, a semiconductor diode detector, a charge coupled device, a vacuum phototube or any detector suitable for use with the linear variable interference filter.

*Alternatively,* the linear variable interference filter may be *operable from a fixed position* on the common optical axis. The advantage of operating the filter from a fixed position on the common optical axis is that the fluorescence light may be spread across the whole filter area, thus allowing the all the fluorescence to be detected at the same time.

Preferably, the apparatus may further comprise a one-dimensional or a two-dimensional array of detectors. The advantage of operating the linear variable interference filter from a fixed position with a one-dimensional or a two-dimensional array of detectors is that the arrays of detectors may be sized to suit the bandwidth of the filter. This is turn may fit the emission bandwidth of the target amino acids of the cataractic eye lens. Another advantage of the one-dimensional or a two-dimensional array of detectors is that the fluorescence spectra may be recorded in a single exposure. Even though this arrangement may exhibit reduced sensitivity, the signal to noise ratio is good enough that this is acceptable for use in treating and monitoring cataracts. Furthermore, this arrangement removes any requirements for moving parts.

Advantageously, the detector employed with the moveable linear variable interference filter is different than any of the detectors employed in the one-dimensional or a two-dimensional array of detectors.

The wavelength selection system may further comprise a phase-sensitive detection system operable at the same pulse frequency as a pulse frequency of the monitoring light source to separate wavelengths of the excited fluorescence light from wavelengths of ambient light. The advantage of employing a phase-sensitive detection system is that the wavelength selection system is operable in near-ambient light conditions by separating fluorescence signals from any stray ambient light and other noise present at different frequencies.

Preferably, the phase-sensitive detection (PSD) system comprises a lock-in amplifier. The advantage of the lock-in amplifier is that the wavelength selection system, by comparing the phases of the reference and incoming signals, may be operable to recover a weak signal from an overwhelming background noise.

Advantageously, the apparatus for monitoring and treating cataracts may be configured to simultaneously monitor cataracts using the monitoring light source and treat cataracts using the treating light source. The simultaneous monitoring and treating of cataracts is advantageously enabled by the optical arrangement of the monitoring light, the treating light and the excited fluorescence light being reflected by the dichroic beam splitter along a common optical axis.

The apparatus for monitoring and treating cataracts may further comprise a *treating dichroic beam splitter* operable to reflect the emitted treating light onto the cataracts. The advantage of employing a treating dichroic beam splitter is that the treating light may be emitted down the same axis as the emitted monitoring light.

Alternatively, the apparatus for monitoring and treating cataracts may further comprise a *MEMS mirror system* operable to move the emitted treating light around various parts of the cataract. The advantage of employing a MEMS mirror system is that the emitted treating light may be moved around the eye to treat all types of cataract. Furthermore, in this arrangement, the apparatus of the present invention may be used with a slit-lamp microscope camera to identify and follow the targeted cataract regions.

In accordance with a *second aspect of the invention,* there is provided a wavelength selection system for use in an apparatus for monitoring and treating cataracts, the wavelength selection system being configured to monitor cataracts by selecting wavelengths of excited fluorescence light in the cataracts. Employing a wavelength selection system in an apparatus for monitoring and treating cataracts has the advantage of allowing the apparatus to become an attachment to existing ophthalmic instruments (such as a slit-lamp microscope), rather than a standalone apparatus. Furthermore, the provision of the wavelength selection system enables the apparatus to provide a comprehensive detection of cataractic changes, even in the early stages of cataract development or as the cataract is reduced because of treatment.

The wavelength selection system may comprise any one or a combination of any one of a *linear variable interference filter, a diffraction grating and* a *refractive prism.* The advantage of such optical elements is that the sensitivity of the apparatus is improved when the apparatus is used to monitor a cataractic eye lens.

Preferably, the linear variable interference filter may comprise a tuneable bandpass interference filter operable in the wavelength range of 320 to 560 nm. The advantage of the chosen bandwidth is that the wavelength selection system allows the apparatus to accurately record without distortion (i.e., with greater sensitivity) the environmentally broadened emissions of tryptophan and NFK emission peaks.

Preferably, the tuneable bandpass interference filter may comprise a wedge filter. The advantage of using a wedge filter as part of the wavelength selection system is that it allows the system to be used for recording non-normal light incidence, which in turn allows a high light grasp, thus making it possible to spectral analyse all the fluorescence emission from the target cataractic eye.

Advantageously, the *wavelength selection system of the second aspect of the invention may be used with the apparatus for monitoring and treating cataracts of the first aspect of the invention.*

In accordance with a *third aspect of the invention*, there is provided a system for use in monitoring and treating cataracts, the system comprising an apparatus for monitoring and treating cataracts and an electronic device, the apparatus comprising a monitoring light source configured to monitor cataracts by emitting monitoring light in the wavelength range of 350 to 410 nm to excite fluorescence in the cataracts, a treating light source configured to treat cataracts by emitting treating light in the wavelength range of 400 to 570 nm to irradiate the cataracts, a wavelength selection system configured to monitor cataracts by selecting wavelengths of the excited fluorescence light in the cataracts and a dichroic beam splitter configured to reflect the monitoring light and the treating light towards the cataracts and the excited fluorescence light in the cataracts towards the wavelength selection system, and the electronic device comprising a data storage and processing device adapted for communication with the wavelength selection system of the apparatus and being configured:
(i) to *manage* the power supply of either or both of the monitoring light source and the treating light source,
(ii) to *control* exposure times for exciting fluorescence light in the cataracts with the monitoring light source
(iii) to *control* exposure times for irradiation of the cataracts with the treating light source, and
(iii) to *select* an operating mode of the apparatus.

Using an electronic device with an apparatus for monitoring and treating cataracts has the advantage of, for example, allowing the operator of the apparatus to be alerted when the catarictic eye moves out of position. Furthermore, the electronic device also allows better control of the actual treatment time and dose of treating light applied by the apparatus.

Preferably, the operating mode of the apparatus may be selected from
- *a monitoring mode* when the electronic device manages and controls the power supply and the exposure time of the monitoring light source or
- *a treatment mode* when the electronic device manages and controls the power supply and the exposure time of the treating light source.

Having the ability to select the operating mode of the apparatus by means of the electronic device has the advantage of allowing the apparatus to select whether the monitoring and treating are undertaken sequentially or simultaneously.

Preferably, the *monitoring mode* of the apparatus may comprise any one or a combination of any one of a *spectral (or full) scan mode or a ratio scan mode.* Being able to select different types of monitoring modes allows the apparatus to comprehensively capture information about the fluorescence spectra of a cataractic lens - operating in a spectral scan mode allows the apparatus to capture the full fluorescence spectrum, whereas operating in a ratio scan mode allows the apparatus to record fluorescence for 2 selected wavelength bands - signal band and reference band - and the electronic device to compute the spectral ratio using the data of each band.

Advantageously, the *system of the third aspect of the invention may comprise the apparatus for monitoring and treating cataracts of the first aspect of the invention.*

### BRIEF DESCRIPTION OF THE DRAWINGS

Various aspects of the invention will now be described by way of example only and with reference to the accompanying drawings, of which:
Figure 1 shows a configuration of the apparatus of the first aspect of the invention;
Figure 2 illustrates a 'ray trace' showing that the LED beam defocuses on the retina and reduces the chance of retinal damage;
Figure 3 shows the spectrum of a tunable bandpass interference filter for use with the apparatus of the first aspect of the invention and the system of the second aspect of the invention;
Figure 4 illustrates the effect on the recorded fluorescence spectrum of a phase-sensitive detection system for use in an embodiment of the apparatus of the first aspect of the invention and in an embodiment of the system of the second aspect of the invention;
Figure 5 shows a configuration of the system of the third aspect of the invention;
Figure 6 illustrates the software architectural design employed by the electronic device of the system of the third aspect of the invention;
Figures 7 shows fluorescence spectra recorded using a first configuration of the apparatus of the first aspect of the invention on a removed pig's lens with excitation at 365nm - fresh lens (dotted line), with UV-induced cataract at 310nm for 2 hours (solid line) and after cataract treatment at 415nm (dashed line);
Figures 8 shows other fluorescence spectra recorded using a second configuration of the apparatus of the first aspect of the invention on a removed pig's lens with excitation at 365nm - fresh lens (dotted line), with UV-induced cataract at 310nm for 2 hours (solid line) and after cataract treatment at 420nm (dashed line);
Figure 9 shows fluorescence spectra recorded using a first configuration of the apparatus of the first aspect of the invention on a live diabetic pig's lens with excitation at 365nm - live cataractic lens (solid line), after cataract treatment at 415nm for 1 hour (dashed line) and after cataract treatment at 415nm for 2 hours (dotted line).

### DETAILED DESCRIPTION OF THE INVENTION

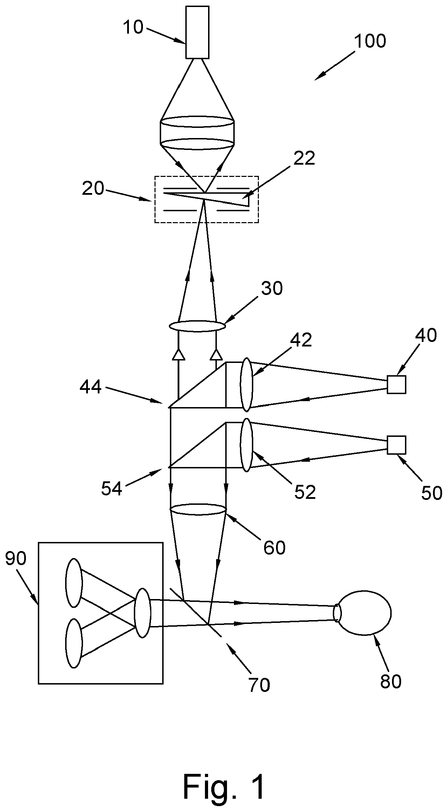

Figure 1 shows a configuration of the apparatus of the first aspect of the invention, the apparatus being used for monitoring and treating cataracts. The core configuration of the apparatus comprises monitoring and treating light sources, a wavelength selection system and a dichroic beam splitter, wherein the monitoring light, the treating light and the excited fluorescence light are reflected by the dichroic beam splitter along a common optical axis. The dichroic beam splitter serves to reflect the monitoring light and the treating light towards the cataracts and the excited fluorescence light in the cataracts towards the wavelength selection system and is arranged at 45 degrees to the common optical axis to transmit wavelengths longer than wavelengths of the monitoring light, the treating light and the excited fluorescence light towards an operator of the apparatus.

In the configuration of Figure 1, the apparatus (100) is shown to comprise:
- a treating light source (40) together with associated optical elements such as a treating focusing lens (42) and a treating dichroic beam splitter (44).

The treating light source (40) is configured to treat cataracts by emitting treating light in the wavelength range of 400 to 570 nm to irradiate the cataracts. The treating light source (40) comprises a non-lasing LED light source operable to emit light in the wavelength range of 400 to 570 nm, preferably in the wavelength range of 410 to 420 nm and more preferably at 415 nm or 420 nm to irradiate the cataracts.

The treating wavelength of 420 nm has been used in pre-clinical trials on removed pig's lenses, whereas the treating wavelength of 415 nm has been used in both pre-clinical trials (on removed pig's lenses and on diabetic live pig's lenses) and in clinical trials.

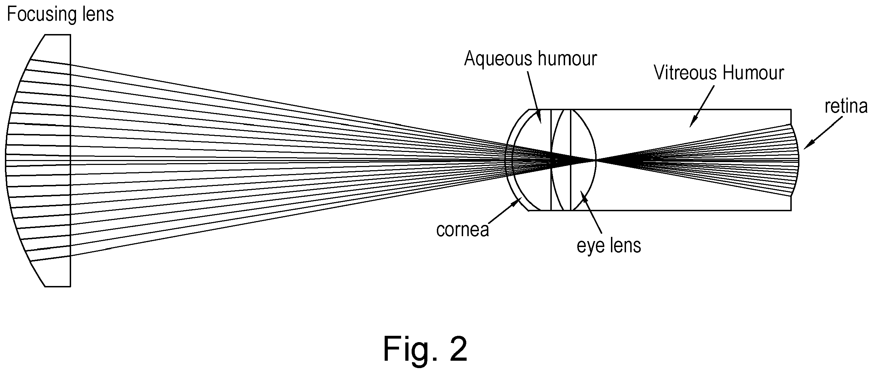

Treating the cataractic lens is also known as photo-bleaching. Using LEDs provides for a non-invasive photo-bleaching treatment that retains the natural lens. A 'ray trace' experiment was carried out to determine the intensity of the treating light at the retina. Figure 2 illustrates a ray trace showing that the LED beam will focus on the lens and defocus over the retina, therefore a reduced power density is seen by the retina compared to the treatment volume in the lens. This, in turn, drastically reduces the chance of retinal damage.

The treating dichroic beam splitter (44) is operable to reflect the emitted treating light onto the cataracts. The treating dichroic beam splitter (44) is a 420/425 nm dichroic and it reflects the treating light at 415 or 420 nm down the common optical axis as the monitoring light, whilst passing wavelengths above 420 nm. This enables the apparatus (100) to simultaneously monitor the treatment effect of the 415nm or 420 nm LED.

Alternatively, a MEMS mirror system (not shown) may be used and is operable to move the emitted treating light around various parts of the cataract to treat all types of cataract. This arrangement requires the use of a slit-lamp microscope camera to enable targeting various parts of the eye with the treating light beam.
- a monitoring light source (50) together with associated optical elements such as a monitoring focusing lens (52) and a treating dichroic beam splitter (54).

The monitoring light source (50) is configured to monitor cataracts by emitting monitoring light in the wavelength range of 350 to 410 nm to excite fluorescence light in the cataracts. The monitoring light source (50) comprises a non-lasing LED light source operable to emit light in the wavelength range of 350 to 410 nm, preferably in the wavelength range of 360 to 370 nm and more preferably at 365 nm to excite fluorescence light in the cataracts. The monitoring wavelength of 365 nm has been used in both pre-clinical trials (on removed pig's lenses and diabetic live pig's lenses) and in clinical trials.

The monitoring dichroic beam splitter (54) is operable to reflect the emitted monitoring light onto the cataracts. The monitoring dichroic beam splitter (54) is a 395 nm dichroic and it reflects the monitoring light at 365 nm down the common optical axis as the treating light, whilst passing wavelengths above 395 nm, including the treating light at 415 nm or 420 nm and the NFK fluorescence peak at around 440 nm. This enables the apparatus (100) to simultaneously monitor the treatment effect of the 415nm or 420 nm LED.
- a wavelength selection system (20) configured to monitor cataracts by selecting wavelengths of the excited fluorescence light in the cataracts.

In the embodiment of Figure 1, the wavelength selection system (20) comprises a wedge filter (22), which is a tuneable bandpass interference filter operable in the wavelength range of 320 to 560 nm.

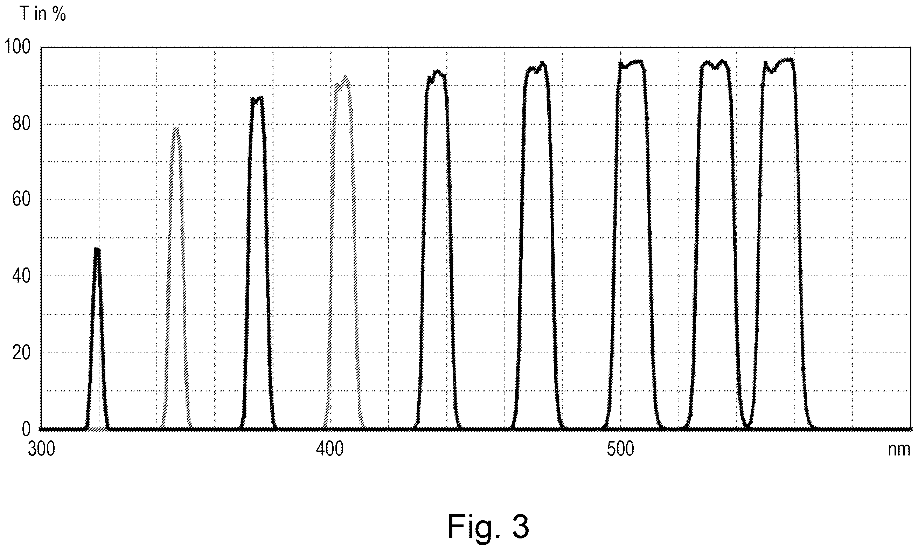

Figure 3 shows the 320 to 560 nm spectrum of the wedge filter (22). The spectral measurements were performed using a 0.2 mm spot width and 1.0 nm spectral bandwidth. The wavelength range of the wedge filter (22) was carefully chosen to allow the environmentally broadened emissions of the tryptophan and NFK to be recorded without distortion. Furthermore, the chosen bandwidth of the wedge filter (22) is wider than what is normal for fluorescence interference filters, therefore providing the wavelength selection system (20) with greater sensitivity in a miniaturised system.

Alternatively, the wavelength selection system (20) may comprise a diffraction grating as an alternative to the wedge filter (22).

The wavelength selection system (20) further comprises *a phase-sensitive detection system* (not shown) operable at the same pulse frequency as a pulse frequency of the monitoring light source (50) to separate wavelengths of the excited fluorescence light from wavelengths of ambient light. The phase-sensitive detection system may be a lock-in amplifier.

The ability to operate the apparatus (100) under ambient lighting conditions will remove the requirement of strictly controlled environmental lighting conditions. This should greatly increase the number of suitable locations where the apparatus (100) can operate.

The phase-sensitive detection system is a system whereby the excitation light from the system is modulated. The system is then able to differentiate between the reflected, modulated light necessary for cataract diagnosis and the ambient, unmodulated light that would otherwise interfere.

An experiment was run to determine the suitability of the phase-sensitive detection (PSD) system in removing ambient light and the following procedure was applied:
- the PSD system was set up with a 365 nm excitation LED targeting a sample of paper
- spectral scans were recorded under the following conditions:
   o Room lights on, PSD system disabled
   o Room lights on, PSD system enabled
   o Room lights off, PSD system disabled
   o Room lights off, PSD system enabled
- the test was carried out in a sealed darkroom.

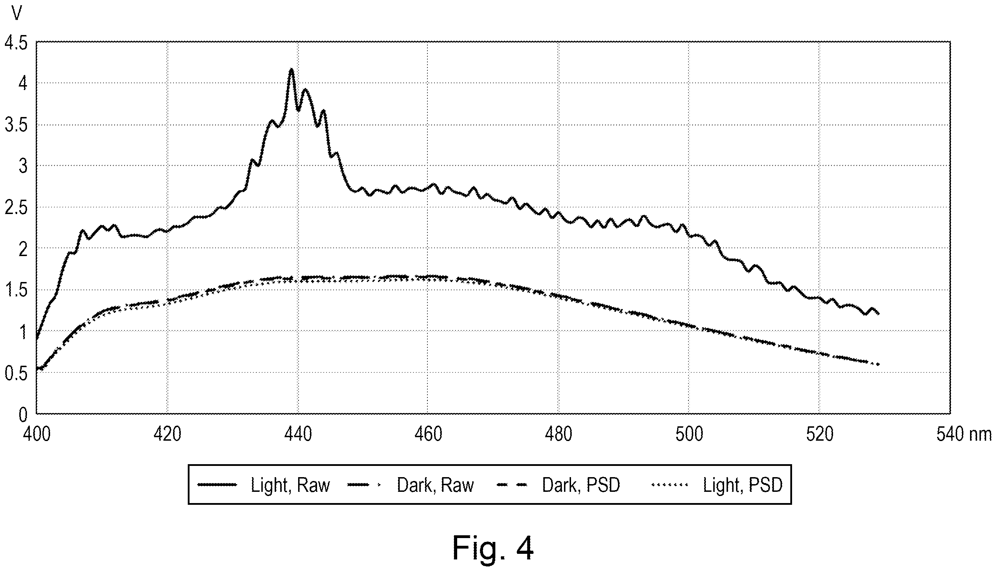

Figure 4 shows the data obtained from the above experiment and illustrates the effect on the recorded fluorescence spectra (400 nm to 530 nm) of a lock-in amplifier employed in an embodiment of the wavelength selection system (20) of the apparatus (100).

The legend in Figure 4 was used as follows:
- the term "PSD" is used when the PSD system was on
- the term "Raw" is used when the PSD system was off
- the term "Light" is used when the lights were on
- the term "Dark" is used when the lights were off

As seen from Figure 4, there is a reduction in signal between the light (solid line) and dark (dot-dashed line) setups with the PSD system off. This indicates that the room lights were contributing to the apparent signal obtained while the PSD system was disabled. When the PSD system was enabled, there is no significant change in signal between the room lights being on (dotted line) and being off (dashed line). Furthermore, the results taken in the dark room especially show that the PSD system outperforms a reasonable attempt to remove ambient lighting.

The data of Figure 4 indicates that the largest change was observed for the 'lights on' set-up when the PSD system was enabled (i.e., switched from off to on). There are two possible sources for this change:
- the first is that modulated light source contains less power than the unmodulated light source as it is being modulated (i.e., effectively turned on and off very quickly). The PSD system is designed to compensate for this modulation and an assumption is made as to how quickly the switching occurs - if the assumption is wrong, then the compensation could be under or overpowered. This compensation however is constant and has no impact on actual measurements made with the PSD system.
- the second is that, while the testing room appeared dark, it was not completely light-tight, thus it is very possible that some light was able to enter the PSD system.

Line frequency of ambient lights varies by country; however, it is generally either 50Hz or 60Hz depending on region. Fluorescent room lighting in the UK shows a peak at 100Hz caused by 50Hz line frequency exciting the tube twice per cycle. An apparent harmonic also appears at 200Hz. Therefore,
- for the 50Hz line frequency used in the UK, a 150Hz PSD system operating frequency is chosen as the centre point between these two peaks, and
- for 60Hz operation the ideal operating frequency of the PSD system is assumed to be 180Hz given similar peaks at 120Hz and 240Hz.

Thus, based on the information above, a PSD system operating frequency of 165Hz should be optimum for both 50Hz and 60Hz line frequencies.

The wavelength selection system (20) further comprises a *linear drive* (not shown) operable to move the linear variable interference filter (wedge filter (22) in Figure 1) along an axis perpendicular to the common optical axis. In this configuration, the detector (10) is a single detector, typically a PMT detector.

Alternatively, the linear variable interference filter (wedge filter (22) in Figure 1) of the wavelength selection system (20) is operable from a fixed position on the common optical axis. In this configuration, the detector (10) is a one-dimensional or a two-dimensional array of detectors (10).
- a dichroic beam splitter (70) configured to reflect the monitoring light and the treating light towards the cataracts and the excited fluorescence light in the cataracts towards the wavelength selection system (20). The monitoring light, the treating light and the excited fluorescence light are reflected by the dichroic beam splitter (70) along a common optical axis.

In the configuration of Figure 1, the dichroic beam splitter (70) is arranged at 45 degrees to the common optical axis to transmit wavelengths longer than wavelengths of the monitoring light, the treating light and the excited fluorescence light towards an operator (not shown) of the apparatus (100). This arrangement is necessary in order to allow the operator to simply swing the apparatus (100) in and out of a slit-lamp microscope (90). In practice, the apparatus (100) is mounted onto a rotation stage with visible markings every 1 degree and a hard stop at the 0-degree (or 'in use') position. This allows the apparatus (100) to be moved out of the way of the slit-lamp (90) and moved back when monitoring or treatment is to be carried out on the eye (80).

The dichroic beam splitter (70) is a 563 nm dichroic and it reflects the required short-wavelength monitoring and treating lights towards the eye (80), whilst allowing long-wavelength visible light to pass through to (i.e., be transmitted towards) the slit-lamp microscope (90) so that a visual (or camera) check on the positioning of the patient's eye (80) can be maintained. This allows the use of a computerised tracking system using the image captured by the camera built into the slip-lamp microscope (90). The tracking software alerts the operator when the eye moves out of position, this in turn, allowing better control of the actual treatment dose for the patient.
- a focusing lens (30) employed to focus the fluorescence light onto the wavelength selection system (20).
- a focusing lens (60) employed used to focus the monitoring light and the treating light onto the dichroic beam splitter (70).

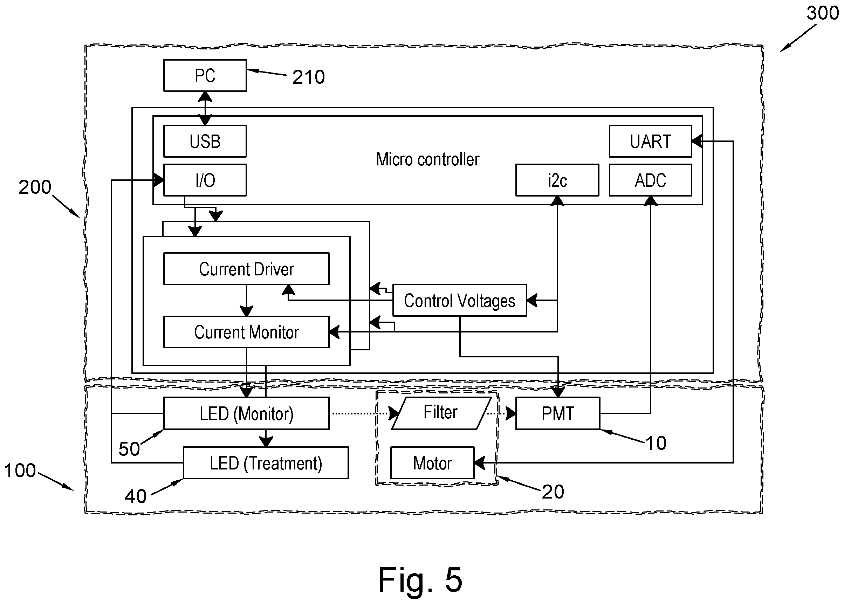

Figure 5 shows a configuration of the system (300) for use in monitoring and treating cataracts, the system (300) being shown to comprise:
- the apparatus (100) of Figure 1 for monitoring and treating cataracts and
- the electronic device (200)

Essential components of the apparatus (100) are also shown in Figure 5, as follows:
- the monitoring light source (50) - labelled as 'LED (Monitor)'
- the treating light source (40) - labelled as 'LED (Treatment)'
- the wavelength selection system (20) with two of its components - the linear variable interference filter (labelled as 'Filter') and the linear drive (labelled as 'Motor')
- the detector (10) - labelled as 'PMT'

The monitoring LED (at 365nm) and the treating LED (at 415 nm or 420 nm) are permanently installed in the system (100) and controlled by bespoke software implemented by the electronic device (200). As each LED has a bandwidth larger than 10 nm, each light beam is filtered by a hard-coated optical filter (not shown) centered near the emission wavelength of each of the LEDs. This reduces unwanted light from entering the apparatus (100). The output of the apparatus (100) to the patient is further filtered by the dichroics (44), (54) and (70) used to fold and direct the internal LED beam paths along a common optical axis. These dichroics are hard coated.

Figure 5 also shows relevant components of the electronic device (200), namely the data storage and processing device (210). The device (210) is adapted for communication with the wavelength selection system (20) of the apparatus (100) and is configured:
(i) to *manage* the power supply of either or both of the monitoring light source (50) and the treating light source (40),
(ii) to *control* exposure times for exciting fluorescence light in the cataracts with the monitoring light source (50)
(iii) to *control* exposure times for irradiation of the cataracts with the treating light source (40), and
(iv) to *select* an operating mode of the apparatus (100).

In use, the apparatus (100) is configured by the electronic device (200) to successively monitor cataracts using the monitoring light source (50) and to treat cataracts using the treating light source (40). Therefore, the operating mode of the apparatus (100) may be selected from
- *a monitoring mode* when the electronic device (200) manages and controls the power supply and the exposure time of the monitoring light source (50) or
- *a treatment mode* when the electronic device (200) manages and controls the power supply and the exposure time of the treating light source (40).

The apparatus (100) is mounted onto a rotation stage (not shown) with visible markings every 1 degree and a hard stop at the 0-degree (or 'in use') position. This allows the apparatus (100) to be moved out of the way of the slit-lamp microscope (90) and moved back when monitoring or treatment is to be carried out on the eye (80).

In *monitoring mode,* the apparatus (100) is used efficiently for cataract assessment by determining fluorescence changes within the cataractous eye (80) caused by the 365 nm monitoring LED (50) exciting the fluorescence within the eye (80). The fluorescence signals return from the patient's eye (80) and are transmitted by the dichroic reflector (70) to give fluorescence spectra at the PMT detector (10).

The spectra are analysed to determine the extent of the patient's cataract. Therefore, through the use of fluorescence spectra, the operator of the apparatus (100) can efficiently and effectively monitor the cataract changes resulting from treatment with the treating light LED (40) of the apparatus (100).

In *treatment mode,* the apparatus (100) focusses the 415nm treating LED (40) onto the patient's cataract for a treatment period of up to 2 hours, which is split into sessions of no longer than 15 minutes per session.

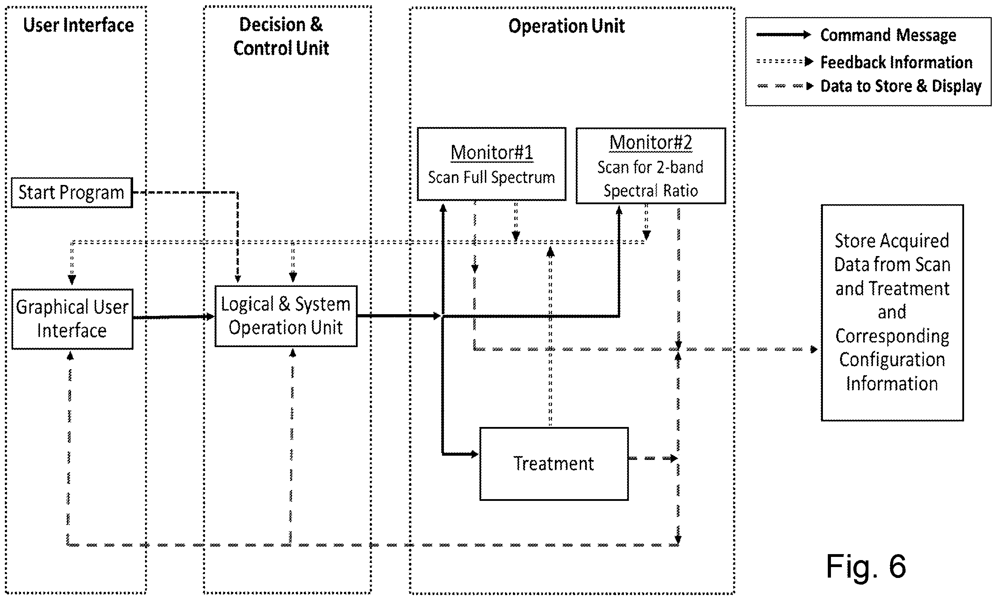

Figure 6 illustrates the software architectural design employed by the electronic device (200) of the system (300). The software firstly establishes a communication channel between the apparatus (100) and the data storage and processing device (210). Using that communication channel, the software can send instructions to the device (210) as well as receive spectral data and the instantaneous status of the apparatus (100). In addition, the software has a control unit which uses the communication channel to control, validate and monitor the sequence of tasks for any operation.

The software has been developed to perform three key operations, as described below:
- *Full Scan Monitor.* Read the wavelength-wise PMT (10) output signal (the Fluorescence strength) and simultaneously draw a wavelength vs Fluorescence strength plot. This plot will provide a spectrum of fluorescence.

For this operation, the software sequentially performs the following tasks:
a) set the intensity and frequency for excitation/monitor LED (50) (wavelength = 365 nm)
b) fix PMT (10) gain voltage for data reading
c) switch the excitation/monitor LED (50) ON,
d) activate the linear motor to move the wedge filter (22) to each nm of wavelength for measurement,
e) simultaneously read, store and display the PMT (10) output signal,
f) switch the excitation/monitor LED (50) OFF
   - *Ratio Scan Monitor*: Read the PMT (10) output signal for 2 selected wavelength bands - signal band and reference band. The spectral ratio is computed using the average data of each band.

For this operation, the software does similar steps as the *Full Scan Monitor* but, in this case, the average value of each band's data and its ratio will be displayed instead of full spectrum.
- *Treatment:* Set the intensity of treatment LED (40) (wavelength = 415nm) and switch on for 15 minutes.

Therefore, the monitoring mode of the apparatus (100) may comprise any one or a combination of any one of a spectral scan mode or a ratio scan mode.

Additionally or alternatively, the apparatus (100) may be configured to simultaneously monitor cataracts using the monitoring light source (50) and treat cataracts using the treating light source (40).

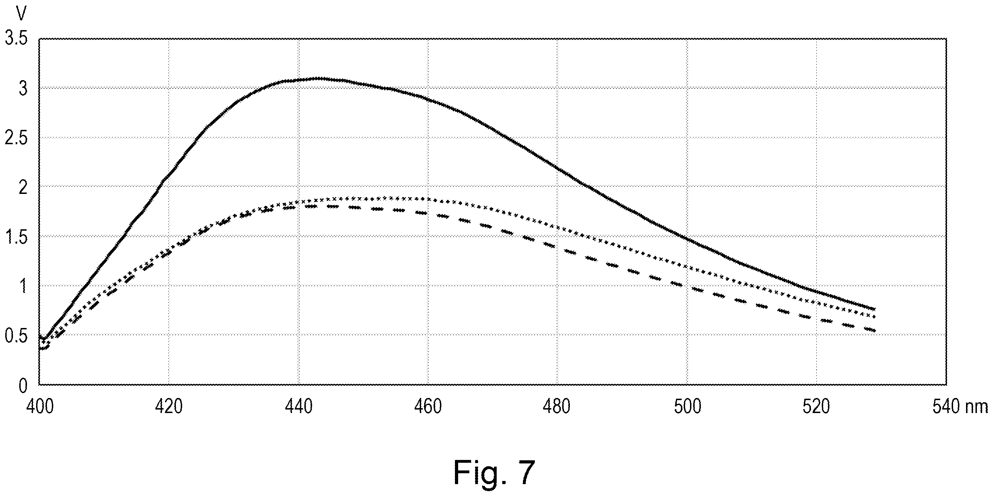

Figure 7 shows fluorescence spectra recorded on a *removed pig's lens* using a first configuration of the apparatus (100). The measurement protocol was such that a cataract was first induced in the lens by irradiating the lens with UV light at 310 nm for 2 hours. The cataract was then treated (or photo-bleached) with treating light at 415 nm. The fluorescence spectra of the different conditions of the removed lens have been recorded with monitoring (or excitation) light at 365 nm and are shown in Figure 7 for - a fresh lens (dotted line), a cataractic lens (solid line) and a treated lens (dashed line). The treated lens displays a fluorescence spectrum very similar to that of the fresh lens.

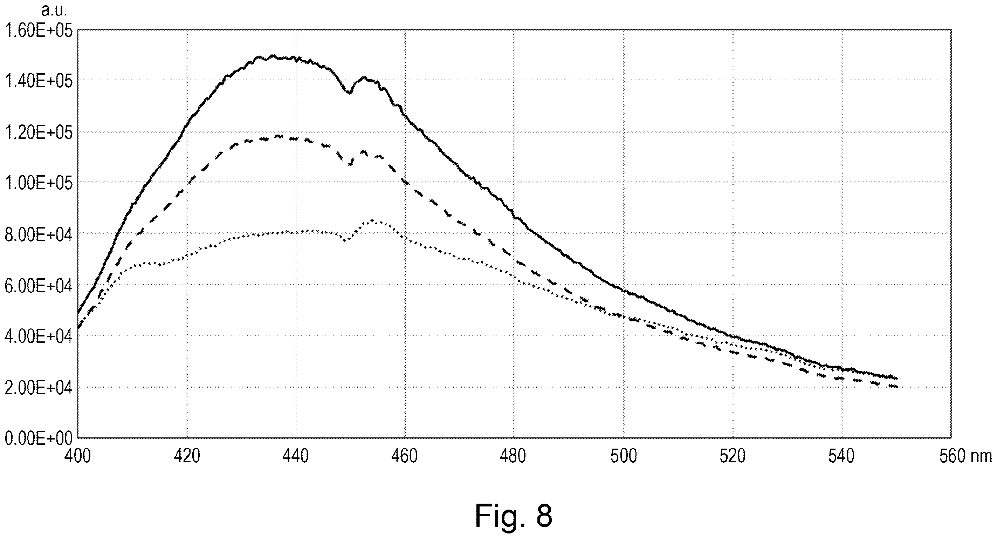

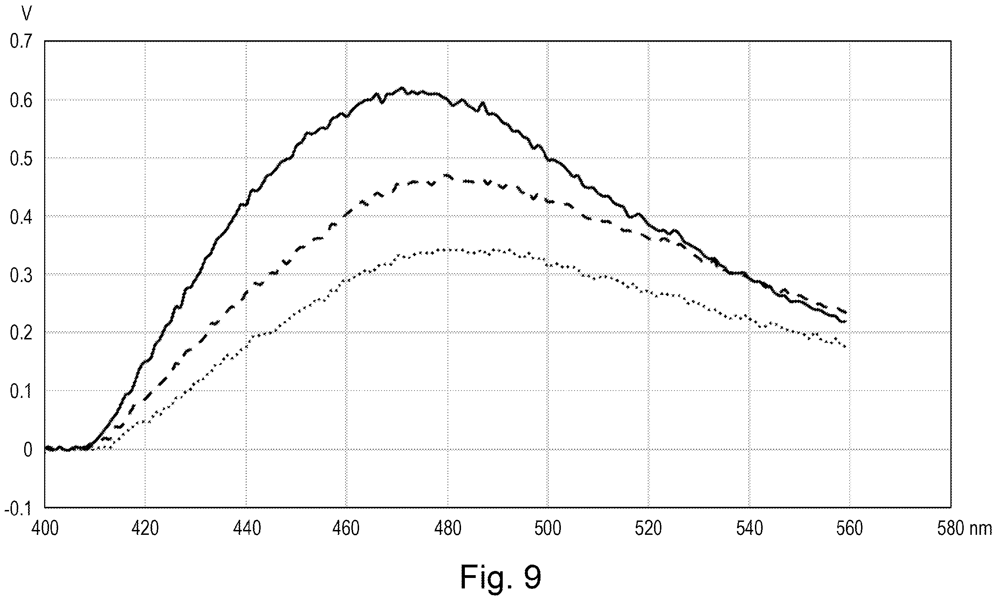

To prove the versatility of the apparatus (100), Figure 8 shows other fluorescence spectra recorded on another *removed pig's lens* using a second configuration of the apparatus (100). As indicated above in relation to Figure 7, the protocol followed was to first induce a cataract in the lens by irradiation at 310 nm for 2 hours. However, in this configuration, the cataract was then treated with treating light at 420 nm for 2 hours. The fluorescence spectra of the different conditions of the removed lens have also been recorded with monitoring light at 365 nm and are shown in Figure 8 for - a fresh lens (dotted line), a cataractic lens (solid line) and a treated lens (dashed line). In this configuration, the fluorescence of the treated lens has been improved as a result of the treatment, but it has not reached that of the fresh lens. Figure 9 shows fluorescence spectra recorded on a *live diabetic pig's lens* using the first configuration of the apparatus (100). The measurement protocol was different because the lens of the live pig was already cataractic since cataracts are one of the sight-related complications of diabetes. Therefore, the cataract was treated with treating light at 415 nm for an initial period of 1 hour, followed by another treatment period of another hour. The fluorescence spectra of the cataractic lens and of the lens treated in two consecutive 1-hour sessions have been recorded with monitoring light at 365 nm and are shown in Figure 9 for - a cataractic live lens (solid line), a lens treated for 1 hour (dashed line) and a lens treated for another hour (dotted line). There is an immediate decrease in the cataract's fluorescence after the first hour of treatment, followed by a consistent decrease following a prolonged treatment period.

Although illustrative embodiments of the invention have been disclosed in detail herein, with reference to the accompanying drawings, it is understood that the invention is not limited to the precise embodiment and that various changes and modifications can be effected therein by one skilled in the art without departing from the scope of the invention as defined by the appended claims and their equivalents. For example, the treating light source (40) and/ or the monitoring light source (50) may comprise a low power laser source or any combination of a polychromatic light source and suitable wavelength selection system.

## Claims

1. An apparatus (100) for monitoring and treating cataracts, the apparatus (100) comprising:
a monitoring light source (50) configured to monitor cataracts by emitting monitoring light in the wavelength range of 350 to 410 nm to excite fluorescence light in the cataracts
a treating light source (40) configured to treat cataracts by emitting treating light in the wavelength range of 400 to 570 nm to irradiate the cataracts
a wavelength selection system (20) configured to monitor cataracts by selecting wavelengths of the excited fluorescence light in the cataracts and
a dichroic beam splitter (70) configured to reflect the monitoring light and the treating light towards the cataracts and the excited fluorescence light in the cataracts towards the wavelength selection system (20)
*wherein* the monitoring light, the treating light and the excited fluorescence light are reflected by the dichroic beam splitter (70) along a common optical axis and
*wherein* the dichroic beam splitter (70) is arranged at 45 degrees to the common optical axis to transmit wavelengths longer than wavelengths of the monitoring light, the treating light and the excited fluorescence light towards an operator of the apparatus (100).

2. The apparatus (100) of claim 1, wherein the monitoring light source (50) comprises a non-lasing LED light source operable to emit light in the wavelength range of 350 to 410 nm, preferably in the wavelength range of 360 to 370 nm and more preferably at 365 nm to excite fluorescence light in the cataracts.

3. The apparatus (100) of any of the preceding claims, wherein the treating light source (40) comprises a non-lasing LED light source operable to emit light in the wavelength range of 400 to 570 nm, preferably in the wavelength range of 410 to 420 nm and more preferably at 415 nm to irradiate the cataracts.

4. The apparatus (100) of any of the preceding claims, wherein the wavelength selection system (20) comprises any one or a combination of any one of a linear variable interference filter, a diffraction grating and a refractive prism.

5. The apparatus (100) of claim 4, wherein the linear variable interference filter (22) comprises a tuneable bandpass interference filter operable in the wavelength range of 320 to 560 nm.

6. The apparatus (100) of claim 5, wherein the tuneable bandpass interference filter comprises a wedge filter (22).

7. The apparatus (100) of any one of claims 4 to 6, wherein the wavelength selection system (20) further comprises a linear drive operable to move the linear variable interference filter along an axis perpendicular to the common optical axis.

8. The apparatus (100) of any one of claims 4 to 7, wherein the apparatus (100) further comprises a detector (10).

9. The apparatus (100) of any one of claims 4 to 6, wherein the linear variable interference filter is operable from a fixed position on the common optical axis.

10. The apparatus (100) of any one of claims 4 to 6 and claim 9, wherein the apparatus (100) further comprises a one-dimensional or a two-dimensional array of detectors (10).

11. The apparatus (100) of any one of claims 4 to 10, wherein the wavelength selection system (20) further comprises a phase-sensitive detection system operable at the same pulse frequency as a pulse frequency of the monitoring light source (50) to separate wavelengths of the excited fluorescence light from wavelengths of ambient light.

12. The apparatus (100) of claim 11, wherein the phase-sensitive detection system comprises a lock-in amplifier.

13. The apparatus (100) of any one of the preceding claims, wherein the apparatus (100) is configured to simultaneously monitor cataracts using the monitoring light source (50) and treat cataracts using the treating light source (40).

14. The apparatus (100) of any of the preceding claims, the apparatus (100) further comprising a treating dichroic beam splitter (44) operable to reflect the emitted treating light onto the cataracts.

15. The apparatus (100) of any one of claims 1 to 13, wherein the apparatus (100) further comprises a MEMS mirror system operable to move the emitted treating light around various parts of the cataract.

16. The apparatus (100) of any of the claims 1-15, wherein the wavelength selection system (20) comprises any one or a combination of any one of a linear variable interference filter, a diffraction grating and a refractive prism.

17. The apparatus (100) of claim 16, wherein the linear variable interference filter comprises a tuneable bandpass interference filter operable in the wavelength range of 320 to 560 nm.

18. The apparatus (100) of claim 17, wherein the tuneable bandpass interference filter comprises a wedge filter (22).

19. A system (300) for use in monitoring and treating cataracts, the system comprising the apparatus (100) for monitoring and treating cataracts of any of the claims 1 to 17, and an electronic device (200), the electronic device (200) comprising a data storage and processing device (210) adapted for communication with the wavelength selection system (20) of the apparatus (100), and being configured:
(i) to *manage* the power supply of either or both of the monitoring light source (50) and the treating light source (40),
(ii) to *control* exposure times for exciting fluorescence light in the cataracts with the monitoring light source (50)
(iii) to *control* exposure times for irradiation of the cataracts with the treating light source (40), and
(iv) to *select* an operating mode of the apparatus (100).

20. The system (300) of claim 19, wherein the operating mode of the apparatus (100) is selected from
- *a monitoring mode* when the electronic device (200) manages and controls the power supply and the exposure time of the monitoring light source (50) or
- *a treatment mode* when the electronic device (200) manages and controls the power supply and the exposure time of the treating light source (40).

21. The system of claim 20, wherein the *monitoring mode* of the apparatus (100) comprises any one or a combination of any one of a spectral scan mode or a ratio scan mode.

## Patentansprüche

1. Vorrichtung (100) zur Überwachung und Behandlung von Katarakten, wobei die Vorrichtung (100) umfasst:
eine Überwachungslichtquelle (50), die konfiguriert ist zum Überwachen von Katarakten durch Emittieren von Überwachungslicht im Wellenlängenbereich von 350 bis 410 nm, um Fluoreszenzlicht in den Katarakten anzuregen
eine Behandlungslichtquelle (40), die konfiguriert ist zum Behandeln von Katarakten durch Emittieren von Behandlungslicht im Wellenlängenbereich von 400 bis 570 nm, um die Katarakte zu bestrahlen
ein Wellenlängenauswahlsystem (20), das konfiguriert ist zum Überwachen von Katarakten durch Auswählen von Wellenlängen des angeregten Fluoreszenzlichts in den Katarakten, und
einen dichroitischen Strahlteiler (70), der konfiguriert ist zum Reflektieren des Überwachungslichts und des Behandlungslichts in Richtung der Katarakte und des angeregten Fluoreszenzlichts in den Katarakten in Richtung des Wellenlängenauswahlsystems (20)
*wobei* das Überwachungslicht, das Behandlungslicht und das angeregte Fluoreszenzlicht von dem dichroitischen Strahlteiler (70) entlang einer gemeinsamen optischen Achse reflektiert werden und
*wobei* der dichroitische Strahlteiler (70) unter 45 Grad zu der gemeinsamen optischen Achse angeordnet ist, um Wellenlängen, die länger sind als Wellenlängen des Überwachungslichts, des Behandlungslichts und des angeregten Fluoreszenzlichts, in Richtung eines Bedieners der Vorrichtung (100) zu übertragen.

2. Vorrichtung (100) nach Anspruch 1, wobei die Überwachungslichtquelle (50) eine nicht-lasernde LED-Lichtquelle umfasst, die betreibbar ist, um Licht im Wellenlängenbereich von 350 bis 410 nm, bevorzugt im Wellenlängenbereich von 360 bis 370 nm und bevorzugter bei 365 nm zu emittieren, um Fluoreszenzlicht in den Katarakten anzuregen.

3. Vorrichtung (100) nach einem der vorhergehenden Ansprüche, wobei die Behandlungslichtquelle (40) eine nicht-lasernde LED-Lichtquelle umfasst, die betreibbar ist, um Licht im Wellenlängenbereich von 400 bis 570 nm, bevorzugt im Wellenlängenbereich von 410 bis 420 nm und bevorzugter bei 415 nm zu emittieren, um die Katarakte zu bestrahlen.

4. Vorrichtung (100) nach einem der vorhergehenden Ansprüche, wobei das Wellenlängenauswahlsystem (20) einen beliebigen oder eine Kombination aus einem beliebigen eines linearen variablen Interferenzfilters, eines Beugungsgitters und eines refraktiven Prismas umfasst.

5. Vorrichtung (100) nach Anspruch 4, wobei der lineare variable Interferenzfilter (22) einen durchstimmbaren Bandpass-Interferenzfilter umfasst, der im Wellenlängenbereich von 320 bis 560 nm betreibbar ist.

6. Vorrichtung (100) nach Anspruch 5, wobei der durchstimmbare Bandpass-Interferenzfilter einen Keilfilter (22) umfasst.

7. Vorrichtung (100) nach einem der Ansprüche 4 bis 6, wobei das Wellenlängenauswahlsystem (20) ferner einen Linearantrieb umfasst, der betreibbar ist, um den linearen variablen Interferenzfilter entlang einer Achse zu bewegen, die senkrecht zur gemeinsamen optischen Achse ist.

8. Vorrichtung (100) nach einem der Ansprüche 4 bis 7, wobei die Vorrichtung (100) ferner einen Detektor (10) umfasst.

9. Vorrichtung (100) nach einem der Ansprüche 4 bis 6, wobei der lineare variable Interferenzfilter von einer festen Position auf der gemeinsamen optischen Achse aus betreibbar ist.

10. Vorrichtung (100) nach einem der Ansprüche 4 bis 6 und Anspruch 9, wobei die Vorrichtung (100) ferner eine eindimensionale oder zweidimensionale Anordnung von Detektoren (10) umfasst.

11. Vorrichtung (100) nach einem der Ansprüche 4 bis 10, wobei das Wellenlängenauswahlsystem (20) ferner ein phasenempfindliches Detektionssystem umfasst, das bei der gleichen Impulsfrequenz wie eine Impulsfrequenz der Überwachungslichtquelle (50) betreibbar ist, um Wellenlängen des angeregten Fluoreszenzlichts von Wellenlängen des Umgebungslichts zu trennen.

12. Vorrichtung (100) nach Anspruch 11, wobei das phasenempfindliche Detektionssystem einen Lock-in-Verstärker umfasst.

13. Vorrichtung (100) nach einem der vorhergehenden Ansprüche, wobei die Vorrichtung (100) konfiguriert ist zum gleichzeitigen Überwachen von Katarakten unter Verwendung der Überwachungslichtquelle (50) und Behandeln von Katarakten unter Verwendung der Behandlungslichtquelle (40).

14. Vorrichtung (100) nach einem der vorhergehenden Ansprüche, wobei die Vorrichtung (100) ferner einen dichroitischen Behandlungsstrahlteiler (44) umfasst, der betreibbar ist, um das emittierte Behandlungslicht auf die Katarakte zu reflektieren.

15. Vorrichtung (100) nach einem der Ansprüche 1 bis 13, wobei die Vorrichtung (100) ferner ein MEMS-Spiegelsystem umfasst, das betreibbar ist, um das emittierte Behandlungslicht um verschiedene Teile des Katarakts herum zu bewegen.

16. Vorrichtung (100) nach einem der Ansprüche 1-15, wobei das Wellenlängenauswahlsystem (20) einen beliebigen oder eine Kombination aus einem beliebigen eines linearen variablen Interferenzfilters, eines Beugungsgitters und eines refraktiven Prismas umfasst.

17. Vorrichtung (100) nach Anspruch 16, wobei der lineare variable Interferenzfilter einen durchstimmbaren Bandpass-Interferenzfilter umfasst, der im Wellenlängenbereich von 320 bis 560 nm betreibbar ist.

18. Vorrichtung (100) nach Anspruch 17, wobei der durchstimmbare Bandpass-Interferenzfilter einen Keilfilter (22) umfasst.

19. System (300) zur Verwendung bei der Überwachung und Behandlung von Katarakten, wobei das System die Vorrichtung (100) zur Überwachung und Behandlung von Katarakten nach einem der Ansprüche 1 bis 17 und ein elektronisches Gerät (200) umfasst,
wobei das elektronische Gerät (200) ein Datenspeicherungs- und -verarbeitungsgerät (210) umfasst, das für die Kommunikation mit dem Wellenlängenauswahlsystem (20) der Vorrichtung (100) ausgelegt und konfiguriert ist:
(i) zum *Verwalten* der Stromversorgung einer oder beider der Überwachungslichtquelle (50) und der Behandlungslichtquelle (40),
(ii) zum *Steuern* von Einwirkungszeiten zur Anregung von Fluoreszenzlicht in den Katarakten mit der Überwachungslichtquelle (50)
(iii) zum *Steuern* von Einwirkungszeiten für die Bestrahlung der Katarakte mit der Behandlungslichtquelle (40) und
(iv) *zum Auswählen* eines Betriebsmodus der Vorrichtung (100).

20. System (300) nach Anspruch 19,
wobei der Betriebsmodus der Vorrichtung (100) ausgewählt ist aus
- einem *Überwachungsmodus,* in dem das elektronische Gerät (200) die Stromversorgung und die Einwirkungszeit der Überwachungslichtquelle (50) verwaltet und steuert, oder
- einem *Behandlungsmodus,* in dem das elektronische Gerät (200) die Stromversorgung und die Einwirkungszeit der Behandlungslichtquelle (40) verwaltet und steuert.

21. System nach Anspruch 20,
wobei der *Überwachungsmodus* der Vorrichtung (100) einen beliebigen oder eine Kombination aus einem beliebigen eines Spektral-Scan-Modus oder eines Verhältnis-Scan-Modus umfasst.

## Revendications

1. Appareil (100) permettant la surveillance et le traitement des cataractes, l'appareil (100) comprenant :
une source de lumière de surveillance (50) configurée pour surveiller les cataractes en émettant une lumière de surveillance dans la plage de longueurs d'onde de 350 à 410 nm pour exciter la lumière de fluorescence dans les cataractes
une source de lumière de traitement (40) configurée pour traiter les cataractes en émettant une lumière de traitement dans la plage de longueurs d'onde de 400 à 570 nm pour irradier les cataractes
un système de sélection de longueur d'onde (20) configuré pour surveiller les cataractes en sélectionnant les longueurs d'onde de la lumière de fluorescence excitée dans les cataractes et
un séparateur de faisceau dichroïque (70) configuré pour réfléchir la lumière de surveillance et la lumière de traitement vers les cataractes et la lumière de fluorescence excitée dans les cataractes vers le système de sélection de longueur d'onde (20)
*dans lequel* la lumière de surveillance, la lumière de traitement et la lumière de fluorescence excitée sont réfléchies par le séparateur de faisceau dichroïque (70) le long d'un axe optique commun et
*dans lequel* le séparateur de faisceau dichroïque (70) est agencé à 45 degrés par rapport à l'axe optique commun pour transmettre des longueurs d'onde plus longues que des longueurs d'onde de la lumière de surveillance, de la lumière de traitement et de la lumière de fluorescence excitée vers un opérateur de l'appareil (100).

2. Appareil (100) de la revendication 1, dans lequel la source de lumière de surveillance (50) comprend une source de lumière LED non laser servant à émettre de la lumière dans la plage de longueurs d'onde de 350 à 410 nm, de préférence dans la plage de longueurs d'onde de 360 à 370 nm et idéalement à 365 nm pour exciter la lumière de fluorescence dans les cataractes.

3. Appareil (100) de l'une quelconque des revendications précédentes, dans lequel la source de lumière de traitement (40) comprend une source de lumière LED non laser servant à émettre de la lumière dans la plage de longueurs d'onde de 400 à 570 nm, de préférence dans la plage de longueurs d'onde de 410 à 420 nm et idéalement à 415 nm pour irradier les cataractes.

4. Appareil (100) de l'une quelconque des revendications précédentes, dans lequel le système de sélection de longueur d'onde (20) comprend l'un quelconque ou une combinaison de l'un quelconque d'un filtre d'interférence variable linéaire, d'un réseau de diffraction et d'un prisme réfractif.

5. Appareil (100) de la revendication 4, dans lequel le filtre d'interférence variable linéaire (22) comprend un filtre d'interférence passe-bande réglable pouvant fonctionner dans la plage de longueurs d'onde de 320 à 560 nm.

6. Appareil (100) de la revendication 5, dans lequel le filtre d'interférence passe-bande réglable comprend un filtre en coin (22).

7. Appareil (100) de l'une quelconque des revendications 4 à 6, dans lequel le système de sélection de longueur d'onde (20) comprend en outre un entraînement linéaire servant à déplacer le filtre d'interférence variable linéaire le long d'un axe perpendiculaire à l'axe optique commun.

8. Appareil (100) de l'une quelconque des revendications 4 à 7, dans lequel l'appareil (100) comprend en outre un détecteur (10).

9. Appareil (100) de l'une quelconque des revendications 4 à 6, dans lequel le filtre d'interférence variable linéaire peut fonctionner à partir d'une position fixe sur l'axe optique commun.

10. Appareil (100) de l'une quelconque des revendications 4 à 6 et de la revendication 9, dans lequel l'appareil (100) comprend en outre un réseau unidimensionnel ou bidimensionnel de détecteurs (10).

11. Appareil (100) de l'une quelconque des revendications 4 à 10, dans lequel le système de sélection de longueur d'onde (20) comprend en outre un système de détection sensible à la phase pouvant fonctionner à la même fréquence d'impulsion qu'une fréquence d'impulsion de la source de lumière de surveillance (50) pour séparer des longueurs d'onde de la lumière de fluorescence excitée de longueurs d'onde de la lumière ambiante.

12. Appareil (100) de la revendication 11, dans lequel le système de détection sensible à la phase comprend un amplificateur de verrouillage.

13. Appareil (100) de l'une quelconque des revendications précédentes, dans lequel l'appareil (100) est configuré pour surveiller simultanément des cataractes à l'aide de la source de lumière de surveillance (50) et traiter des cataractes à l'aide de la source de lumière de traitement (40).

14. Appareil (100) de l'une quelconque des revendications précédentes, l'appareil (100) comprenant en outre un séparateur de faisceau dichroïque de traitement (44) servant à réfléchir la lumière de traitement émise sur les cataractes.

15. Appareil (100) de l'une quelconque des revendications 1 à 13, dans lequel l'appareil (100) comprend en outre un système de miroir MEMS servant à déplacer la lumière de traitement émise autour de diverses parties de la cataracte.

16. Appareil (100) de l'une quelconque des revendications 1 à 15, dans lequel le système de sélection de longueur d'onde (20) comprend l'un quelconque ou une combinaison de l'un quelconque d'un filtre d'interférence variable linéaire, d'un réseau de diffraction et d'un prisme réfractif.

17. Appareil (100) de la revendication 16, dans lequel le filtre d'interférence variable linéaire comprend un filtre d'interférence passe-bande réglable pouvant fonctionner dans la plage de longueurs d'onde de 320 à 560 nm.

18. Appareil (100) de la revendication 17, dans lequel le filtre d'interférence passe-bande réglable comprend un filtre en coin (22).

19. Système (300) destiné à être utilisé dans la surveillance et le traitement des cataractes, le système comprenant l'appareil (100) permettant la surveillance et le traitement des cataractes de l'une quelconque des revendications 1 à 17, et un dispositif électronique (200),
le dispositif électronique (200) comprenant un dispositif de stockage et de traitement de données (210) adapté pour communiquer avec le système de sélection de longueur d'onde (20) de l'appareil (100), et étant configuré :
(i) pour *gérer* l'alimentation électrique de l'une ou des deux sources de lumière de surveillance (50) et de traitement (40),
(ii) pour *réguler* des temps d'exposition pour exciter la lumière de fluorescence dans les cataractes avec la source de lumière de surveillance (50)
(iii) pour *réguler* des temps d'exposition pour l'irradiation des cataractes avec la source de lumière de traitement (40), et
(iv) pour *sélectionner* un mode de fonctionnement de l'appareil (100).

20. Système (300) de la revendication 19,
dans lequel le mode de fonctionnement de l'appareil (100) est choisi parmi
- un *mode de surveillance* lorsque le dispositif électronique (200) gère et régule l'alimentation électrique et le temps d'exposition de la source de lumière de surveillance (50) ou
- un *mode de traitement* lorsque le dispositif électronique (200) gère et régule l'alimentation électrique et le temps d'exposition de la source de lumière de traitement (40).

21. Système de la revendication 20,
dans lequel le *mode de surveillance* de l'appareil (100) comprend l'un quelconque ou une combinaison de l'un quelconque d'un mode de balayage spectral ou d'un mode de balayage de rapport.
